# EUROPEAN PATENT SPECIFICATION

(11) **EP 4 327 748 B1**
(45) Date of publication and mention of the grant of the patent: **10.12.2025**
(21) Application number: 22191668.7
(22) Date of filing: 23.08.2022
(51) Int. Cl.: A61B 6/03, A61B 5/055, A61B 6/04, A61B 6/00

(54) **DRAPING A WALL OF AN IMAGING DEVICE**
DRAPIEREN EINER WAND EINER BILDGEBUNGSVORRICHTUNG
DRAPAGE D'UNE PAROI D'UN DISPOSITIF D'IMAGERIE

(43) Date of publication of application: 28.02.2024
(73) Proprietor: Siemens Healthineers AG, 91301 Forchheim (DE); The Regents of the University of Michigan, Ann Arbor, MI 48109 (US)
(72) Inventor: Gulani, Vikas, Shaker Heights (US); Hengerer, Arne, 91096 Möhrendorf (DE)
(74) Representative: Siemens Healthineers Patent Attorneys

(56) References cited:
- DE-A1- 102004 007 427
- US-A1- 2008 216 844
- US-A1- 2015 114 404
- US-A1- 2020 054 299

## Description

The present invention relates to a device for imaging at least a part of a patient including detection means, which comprises a tunnel (also called bore) through which or into which the patient can be transported for imaging purposes. Furthermore, the present invention relates to a method of covering or draping a tunnel of the mentioned device.

In minimally invasive medical procedures, medical instruments, such as catheters and/or single-use needles, are inserted into a patient, usually with image monitoring of the procedure. Image monitoring allows the acquisition of images in which the medical instrument is visualized in relation to its anatomical surroundings. While classically X-ray imaging has been used for image monitoring of minimally invasive medical procedures, particularly fluoroscopy, it has now also been proposed to use magnetic resonance devices, hence magnetic resonance (MR) imaging, for image monitoring. This is typically referred to as interventional MR imaging. When using so-called closed magnetic resonance devices, which have a main magnet with a cylindrical patient receiving area in which the homogeneity volume is located, it is necessary to work in a rather confined space, which should be sterile for the intervention.

A special type of medical instruments that are often used for minimally invasive medical procedures are interventional needles, which are used, for example, for biopsy, ablation or brachytherapy. It has also been proposed to propagate needles under real-time magnetic resonance control.

It is a common challenge to maintain a sterile field when performing image-guided intervention in both CT (computer tomography) and MRT (magnetic resonance tomography) and any other potential setting with a bore or tunnel through which the patients are put into or pulled out before and after the procedure. In such procedures, needles, biopsy guns, introducers, wires or other devices are partially in the patient and partially outside. Other materials such as insterile towels or drapes may be resting on the patient. When the patient is moved into or out from the imaging device (scanner) on a moving table, there is a risk that these insterile materials contact the device bore, thereby breaking sterility. This may result in the need to dispose of the equipment, repeat parts of the procedure or, in the worst case, abort the procedure.

From document US 2008/216844 A1 a sterile drape for use with a magnet of a magnetic resonance imaging is known which is formed from a generally cylindrical sheet of a fabric. This is collapsible into an annulus and can be expanded axially to line the interior of the bore of the magnet. End flaps can close the ends of the bore and folded outwardly to cover the end faces of the magnet. The sheet can be supported by a wire or other support and can be stored on the magnet and end covers or in a ceiling suspended container.

Document US 2020/054299 A1 discloses an imaging system gantry patient channel drape which includes a gantry first outer sidewall covering portion including an outer rim biased into a deployed (e.g., circular) shape and collapsible, and a gantry inner (e.g., circular) wall covering portion extending inward of the gantry first outer sidewall covering portion.

From document DE 10 2004 007427 A1 an instrument is known which has an examination or therapy area with a wall, and a patient couch table with a horizontally movable patient couch. The couch partly slides inside the area for examining a patient lying on the couch. An inner side of the area is provided with a covering unit made of a fabric, which covers a part of the wall surface. The shape of the unit is partly adapted to the shape of the wall. The covering unit is also made of cellulose e.g. flax, hemp, cotton, paper, velvet, wool, silk and fur. The covering can be optically or haptically changed.

Thus, the object of the present invention is to provide a device for imaging at least a part of a patient which reduces the risk of breaking sterility.

This object is solved by a device and a method according to the independent claims. Favorable further developments are defined in the sub claims.

Accordingly, there is provided a device for imaging at least a part of a patient including detection means comprising a tunnel through which or into which the patient can be transported for imaging purposes. For instance, such device is a CT or MRT equipment for performing image guided intervention. Such CT or MRT device typically has a tunnel or bore for the patient to be screened. Usually, the patient is placed on a patient table which can be moved forward and backward along its longitudinal axis. This patient table allows the patient to be pushed or automatically moved into the tunnel of the imaging device. The tunnel represents an examination area in which the patient or a part of the patient can be screened.

The inventive device includes a sterile cover rolled up or folded at the outside of the tunnel. Preferably, the sterile cover is provided directly on the outside of the tunnel. The sterile cover may be made of any kind of foil, fabric, film or tissue that is sterile itself. A paper or a plastic film may form this cover, being rolled up or folded before use. For example, the cover is rolled up on a stick or a paper roll. Alternatively, the cover is exactly folded or just unsystematically compressed at the outside of the tunnel. Since the cover is provided at the outside of the tunnel, it may be applied easily in the tunnel by pulling it through the tunnel. The device is not limited to one single sterile cover being provided at the outside of the tunnel. Rather a plurality of sterile covers may be provided there in a rolled up or folded state.

The device is provided with a covering mechanism capable of unrolling or unfolding the sterile cover and draping it in the tunnel, such that the inner wall of the tunnel is at least partly covered by the sterile cover.

The covering mechanism includes umbrella-like means for unfolding the sterile cover and for pressing the sterile cover to the inner wall of the tunnel, when the umbrella-like means is pushed through the sterile cover in the tunnel. For instance, when the cover has a tube shape in the unfolded state, it may be flat when folded. In order to unfold such flat cover, it may be necessary to press the cover from the inside out. Such radial pressing may be performed by an umbrella-like tool. The tip of the umbrella opens the folded cover and the distal ends of the umbrella press the cover towards the inner wall of the tunnel.

The goal is to provide a sterile environment for each image guided intervention. Thus, it is essential to cover at least a part of the tunnel or to cover at least the intervention area so that a part of the tunnel or the complete tunnel is protected.

In a preferred embodiment of the inventive device the detection means is based on MRT technology or CT technology. As indicated above these technologies usually include imaging sections having a tunnel or bore which has to be covered by the sterile cover. Additionally, these technologies are preferred for performing image guided interventions.

A further favorable embodiment of the inventive device includes a table for transporting the patient into the tunnel, wherein the table can be moved into the tunnel being covered by the sterile cover without damaging it. During transport into the screening area, i.e. the bore or tunnel, of the scanner the patient may lie on the table. The table may be moved automatically into the tunnel. Thus, the table being moved shall not damage or destroy the sterile cover. This may require that the table is not supported in the tunnel. The table rather has to be supported outside the tunnel whereby the table forms a kind of cantilever. This cantilever may be moved linearly into the tunnel and out of it.

According to a further embodiment it is provided that the sterile cover has a cylindrical or tube-like shape with a diameter of the order of the tunnel. For example, the sterile cover is a plastic film tube. The tube should roughly have a diameter similar to that of the inner wall of the tunnel. Thus, the already small diameter of the tunnel is not lowered by the tube-like cover.

Furthermore, the sterile cover may be one of a series of covers each separated from the other by a respective perforation. In this case, a plurality of covers is fixed together in order to form a structure of a large number of covers. This structure should also be folded or rolled up at the outside of the tunnel. The covering mechanism may be able to separate one cover from the structure of covers by cutting the perforation. Alternatively, the perforation may be cut by a user and the covering mechanism just drapes the separated cover in the tunnel.

Preferably, the sterile cover and/or the inner wall of the tunnel includes sticky material to fix the sterile cover at the inner wall of the tunnel. The sticky material may include glue or a velco fastener or the like. The sticky material guarantees that the cover remains fastened at the inner wall of the tunnel during the intervention. Preferably, the glue or adhesive is completely removed from the inner wall when the sterile cover originally provided with the glue is detached from the inner wall. Otherwise, when the sticky material is provided originally at the inner wall, detachment of the sterile cover should not lead to a tearing of the cover.

As already indicated above, the sterile cover can be largely made of paper or a plastic film. Such paper or plastic film are cheap materials predestinated for single use. The paper may be made of multi-layer material. Furthermore, the material of the sterile cover may be a composite material including paper and a plastic film. Moreover, the paper may also be laminated or sprayed with plastic.

According to the present invention, there is also provided a system for performing an image guided intervention, including a device for imaging as described above and including an intervention device. Such intervention device may be an ablation needle, a biopsy gun, an introducer, a guiding wire or the like. Each of these intervention devices will be contaminated when used for the intervention. The intervention device may touch the inner wall of the tunnel of the imaging device so that sterility is broken. For avoiding such situation, the tunnel of the imaging device or the intervention device itself is covered by the sterile cover.

Furthermore, according to the present invention there is provided a method of covering a tunnel of a device, wherein the covering mechanism includes umbrella-like means for unfolding the sterile cover and for pressing the sterile cover to the inner wall of the tunnel then the umbrella-like means is pushed through the sterile cover in the tunnel. The method comprises the steps of providing the sterile cover having a cylindrical shape at a first axial end of the tunnel and both unfolding and pressing the sterile cover to the inner wall of the tunnel by pushing the umbrella-like means through the sterile cover in the tunnel. The method can be complimented and varied according to the above-described device.

The advantages of the magnetic resonance apparatus according to the invention essentially correspond to the advantages of the method according to the invention, which are described in detail above. Features, advantages or alternative embodiments mentioned herein can likewise be transferred to the other claimed objects and vice versa.

Further advantages, features and details of the invention will be apparent from the examples of embodiments described below and from the drawings.

For use cases or use situations which may arise in the method and which are not explicitly described here, it may be provided that, in accordance with the method, an error message and/or a prompt for user feedback is output and/or a default setting and/or a predetermined initial state is set.

The present invention will be described in more detail along with the following figures showing:
- FIG 1: an MRT system according to the prior art;
- FIG 2: a sectional view of a magnetic resonance device with controlling components as block diagram;
- FIG 3: a cross-sectional view of the magnetic unit of an MRT-device with a sterile cover and a covering mechanism; and
- FIG 4: a schematic block diagram showing an embodiment of the inventive method.

The following embodiments described in more detail are preferred examples of the present invention.

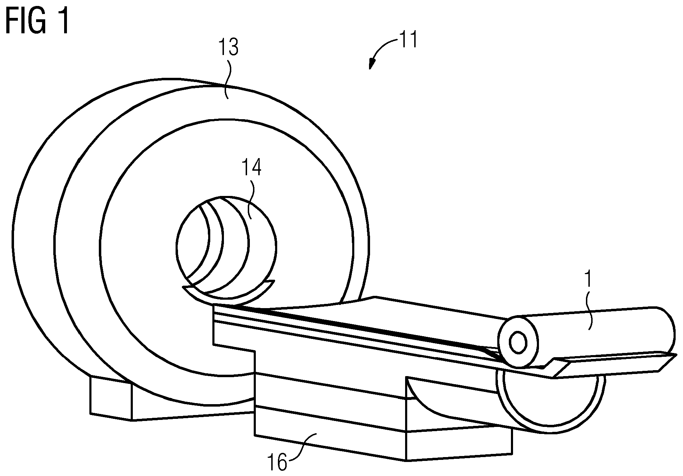

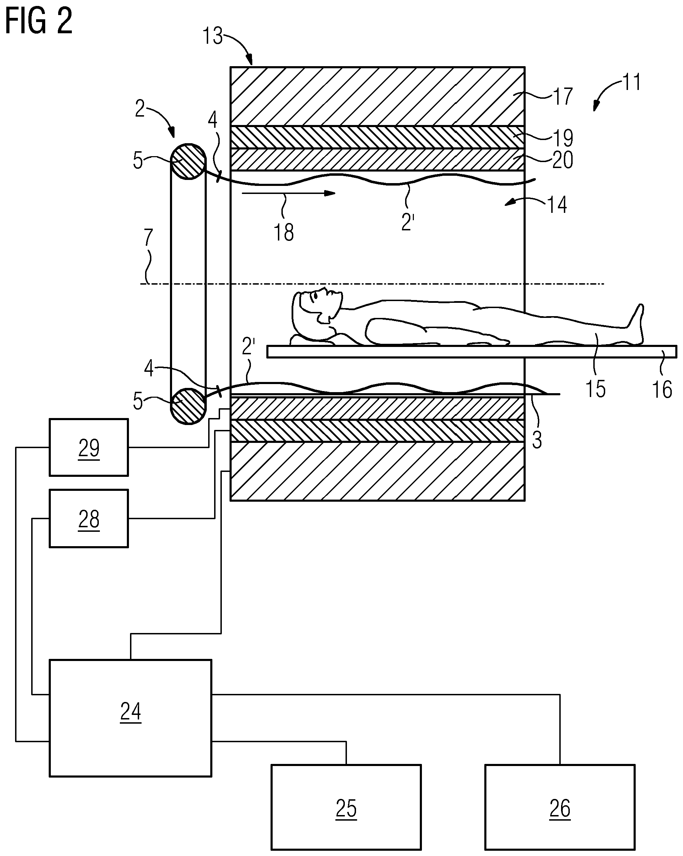

FIG 1 and 2 show a magnetic resonance device 11 (MRT device) as imaging device for performing an image guided intervention in a schematic representation.

The perspective view of FIG 1 shows the magnetic resonance device 11 including the magnet unit 13 as detection means for gathering images of a patient or a part of a patient. For this purpose, the patient has to be introduced into a tunnel 14 of the magnet unit 13. A patient table 16 is provided in front of the magnet unit 13 on which the patient can lie down. The patient table 16 may be designed to position the patient in the tunnel 14 (semi-) automatically.

In order to guarantee sterility of the patient table 16 a paper roll 1 is provided at the foot of the patient table 16. The paper of the paper roll 1 can be pulled over the lying surface of the patient table 16.

As shown in FIG 2 the magnetic resonance apparatus 11 comprises detection means formed by the magnet unit 13 with a main magnet 17 for generating a strong and, in particular, constant main magnetic field 18. In addition, the magnetic resonance apparatus 11 has the cylindrical patient-receiving tunnel 14 for receiving a patient 15, the patient-receiving tunnel 14 being surrounded in a cylindrical manner in a circumferential direction by the magnet unit 13. The patient 15 can be pushed into the patient receiving tunnel 14 by means of the patient table 16 of the magnetic resonance apparatus 11.

The magnet unit 13 further comprises a gradient coil unit 19 used for location coding during imaging. The gradient coil unit 19 is controlled by a gradient control unit 28. Further, the magnet unit 13 has a high-frequency antenna unit 20 and a high-frequency antenna control unit 29 for exciting a polarization established in the main magnetic field 18 generated by the main magnet 17. The high-frequency antenna unit 20 is controlled by the high-frequency antenna control unit 29 and irradiates high-frequency pulses into an examination space substantially formed by the patient receiving tunnel 14.

For controlling the main magnet 17, the magnetic resonance apparatus 11 includes a control unit 24. The control unit 24 centrally controls the magnetic resonance apparatus 11, such as performing MR control sequences. The control unit 24 together with the magnetic unit 13 may be referred to as the recording unit or detection means. In addition, the control unit 24 includes an unspecified reconstruction unit for reconstructing medical image data acquired during the magnetic resonance examination. The magnetic resonance apparatus 11 comprises a display unit 25. Control information, such as control parameters, as well as reconstructed image data may be displayed on the display unit 25, for example on at least one monitor, for a user. In addition, the magnetic resonance apparatus 11 has an input unit 26 by means of which information and/or control parameters can be input by a user during a measurement process. The control unit 24 may comprise the gradient control unit 28 and/or the high-frequency antenna control unit 29 and/or the display unit 25 and/or the input unit 26.

The control unit 24 may comprise computer programs and/or software, which are directly loadable in an unspecified memory unit of the control unit 24 for controlling covering mechanism. For this purpose, the control unit 24 may comprise a processor, not shown in more detail, which is adapted to execute the computer programs and/or software. Alternatively, the computer programs and/or software can also be stored on an electronically readable data carrier formed separately from the control unit 24, whereby data access from the control unit 24 to the electronically readable data carrier can take place via a data network.

The magnetic resonance device 11 shown can of course comprise further components which magnetic resonance devices usually have. Moreover, a general mode of operation of a magnetic resonance apparatus 11 is known to those skilled in the art, so that a detailed description of the further components is dispensed with.

During an invention the MRT device 11 may be contaminated by the patient, by the instruments used, by the physician and the like. The main idea of the present invention therefore is to avoid contamination by draping the bore or tunnel 14 of the imaging device. In FIG 2 the principal means for realizing this draping of tunnel 14 are shown schematically. First of all, a sterile cover 2 is provided at the entrance of tunnel 14, i.e. outside of the tunnel 14. The sterile cover 2 is provided in a rolled up or folded state at the outside of the tunnel 14.

A covering mechanism 3 can be provided in tunnel 14 or outside of tunnel 14. The function of covering mechanism 3 is to pull the sterile cover 2 through the tunnel 14 thereby unrolling or unfolding the sterile cover. The unrolled or unfolded sterile cover 2' is shown in FIG 2 in the tunnel 14. The unfolded sterile cover 2' in the tunnel may be separated by a perforation 4 from other sterile covers 2 folded outside the tunnel 14. The sterile cover 2, 2' alleviates or avoids the problem of contamination of the bore or imaging device.

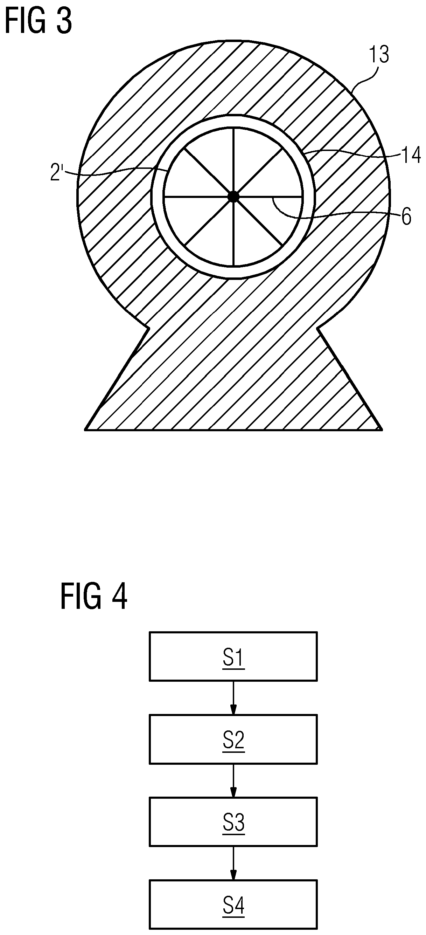

In the following some embodiments of draping the bore of the device with a sterile material are described. According to a first embodiment, the sterile cover 2 is formed like a tube. It may be folded in the shape of a ring 5 and positioned at the entrance of tunnel 14. The covering mechanism 3 for unrolling or unfolding the sterile cover 2 includes an umbrella-like device 6 as shown in the cross-sectional view of the magnet unit 13 in FIG 3. The draping of the tunnel 14 is performed before the patient 15 is pushed into tunnel 14. The tube-like sterile cover 2' shall be spread in tunnel 14. For this purpose, the umbrella-like device 6 is introduced into the cover tube in order to spread it in tunnel 14. In a first variation, the umbrella-like device has fixed arms which spread the cover 2' towards the tunnel wall. According to another variation of the umbrella-like device 6, its arms are movable and are spread within the sterile cover 2' in tunnel 14, thereby pressing the sterile cover 2' towards the inner wall of the tunnel 14.

The plastic foil or paper of the sterile cover 2, 2' may have glue or other sticky material at its surface to hold it against the inner wall of the tunnel. Alternatively, there could be sticky material on the inner wall of the tunnel to detachable fix the cover to the outside of the bore. Furthermore, there could be sticky material on the outside of the covering, to stick a part of the covering to the outside of the bore in such a way that the bore itself is covered.

In another embodiment the imaging device, e.g. the MRT-device 11, is always dedicated to intervention. In this case storage means can be associated with the bore of the imaging device on one or both sides. This storage means (one possible realization is the ring 5 in FIG 2) releases the plastic or paper cover 2 that can be pulled through to the other side of tunnel 14 and attached to the scanner.

All the above embodiments allow for protecting the inside of the scanner so that accidental or minor contact of interventional equipment with the bore does not break the sterile field.

One possible embodiment of the inventive method is now described in connection with FIG 4. In a first step S1 the sterile cover is provided at the outside of the bore 14 of the imaging device 11. The sterile cover 2, 2' may be provided in a folded or rolled state on the one hand or in a unfolded or unrolled state on the other hand.

In a second step S2 the sterile cover 2, 2' is pulled into the bore. If necessary, the sterile cover 2, 2' is unfolded or unrolled thereby. This step of pulling may be performed by the covering mechanism 3. The covering mechanism may include a linear transport system in parallel to the center axis 7 of the bore and grabbing means for grabbing the sterile cover 2, 2'.

In a further optional step S3 the sterile cover 2, 2' is pressed against the inner wall of the bore 14. This pressing can be performed by the covering mechanism, 2. The covering mechanism includes the umbrella-like device as described above.

In a further optional step S4 the sterile cover 2, 2' is stuck or tacked to the inner wall of the bore 14. A suitable glue may be used for this purpose. Alternatively, if the cover should be used multiply, disinfecting it after each use, Velcro fastener can be used for tacking the cover to the inner wall of the bore. Even other fasteners may be used.

Although the invention has been illustrated and described in more detail by the preferred embodiments, the invention is not limited by the disclosed examples and other variations may be derived therefrom by those skilled in the art without departing from the scope of protection of the invention.

## Claims

1. Device (11) for imaging at least a part of a patient (15), including
- detection means (13) comprising a tunnel (14) through which or into which the patient (15) can be transported for imaging purposes,
- a sterile cover (2, 2') rolled up or folded at the outside of the tunnel (14) and
- a covering mechanism (3) capable of unrolling or unfolding the sterile cover (2, 2') and draping it in the tunnel (14), such that the inner wall of the tunnel (14) is covered at least partly by the sterile cover (2, 2'),
**characterized by**
the covering mechanism (3) including umbrella-like means (6) for unfolding the sterile cover (2, 2') and for pressing the sterile cover (2, 2') to the inner wall of the tunnel (14) when the umbrella-like means (6) is pushed through the sterile cover (2, 2') in the tunnel (14).

2. Device (11) according to claim 1, wherein the detection means is based on MRT technology or CT technology.

3. Device (11) according to claim 1 or 2, further including a table (16) for transporting the patient (15) into the tunnel (14), wherein the table (16) can be moved into the tunnel (14) being covered by the sterile cover (2, 2') without damaging it.

4. Device (11) according to one of the preceding claims, wherein the sterile cover (2, 2') has a cylindrical shape with a diameter of the order of the tunnel (14).

5. Device (11) according to claim 4, wherein the sterile cover (2, 2') is one of a series of covers each separated from the other by a respective perforation (4).

6. Device (11) according to one of the preceding claims, wherein the sterile cover (2, 2') and/or the inner wall of the tunnel (14) includes sticky material to fix the sterile cover (2, 2') at the inner wall of the tunnel (14).

7. Device (11) according to one of the preceding claims, wherein the sterile cover (2, 2') is largely made of paper or a plastic film.

8. System for performing an image guided intervention, including a device (11) for imaging according to one of the preceding claims and an intervention device.

9. Method of covering a tunnel (14) of a device (11) according to claim 1, comprising the steps of
- providing the sterile cover (2, 2') having cylindrical shape at a first axial end of the tunnel (14) and
- both unfolding and pressing the sterile cover (2, 2') to the inner wall of the tunnel (14) by pushing the umbrella-like means (6) through the sterile cover (2, 2') in the tunnel (14).

## Patentansprüche

1. Vorrichtung (11) zum Abbilden mindestens eines Teils eines Patienten (15), aufweisend
- ein Detektionsmittel (13), das einen Tunnel (14) umfasst, durch den oder in den der Patient (15) zu Abbildungszwecken transportiert werden kann,
- eine sterile Abdeckung (2, 2'), die an der Außenseite des Tunnels (14) aufgerollt oder zusammengefaltet ist, und
- einen Abdeckmechanismus (3), der die sterile Abdeckung (2, 2') entrollen oder entfalten und den Tunnel (14) innen damit behängen kann, so dass die Innenwand des Tunnels (14) zumindest teilweise von der sterilen Abdeckung (2, 2') abdeckt ist,
**dadurch gekennzeichnet, dass**
der Abdeckmechanismus (3) ein schirmartiges Mittel (6) zum Entfalten der sterilen Abdeckung (2, 2') und zum Andrücken der sterilen Abdeckung (2, 2') an die Innenwand des Tunnels (14), wenn das schirmartige Mittel (6) durch die sterile Abdeckung (2, 2') in dem Tunnel (14) geschoben wird, aufweist.

2. Vorrichtung (11) nach Anspruch 1, wobei das Detektionsmittel auf MRT-Technologie oder CT-Technologie basiert.

3. Vorrichtung (11) nach Anspruch 1 oder 2, ferner aufweisend einen Tisch (16) zum Transportieren des Patienten (15) in den Tunnel (14), wobei der Tisch (16) in den von der sterilen Abdeckung (2, 2') abgedeckten Tunnel (14) bewegt werden kann, ohne diese zu beschädigen.

4. Vorrichtung (11) nach einem der vorhergehenden Ansprüche, wobei die sterile Abdeckung (2, 2') eine zylindrische Form mit einem Durchmesser in der Größenordnung des Tunnels (14) aufweist.

5. Vorrichtung (11) nach Anspruch 4, wobei die sterile Abdeckung (2, 2') eine aus einer Reihe von Abdeckungen ist, die jeweils durch eine jeweilige Perforation (4) voneinander getrennt sind.

6. Vorrichtung (11) nach einem der vorhergehenden Ansprüche, wobei die sterile Abdeckung (2, 2') und/oder die Innenwand des Tunnels (14) klebriges Material zum Fixieren der sterilen Abdeckung (2, 2') an der Innenwand des Tunnels (14) aufweist.

7. Vorrichtung (11) nach einem der vorhergehenden Ansprüche, wobei die sterile Abdeckung (2, 2') weitgehend aus Papier oder einer Kunststofffolie hergestellt ist.

8. System zum Durchführen eines bildgeführten Eingriffs, aufweisend eine Abbildungsvorrichtung (11) nach einem der vorhergehenden Ansprüche und eine Eingriffsvorrichtung.

9. Verfahren zum Abdecken eines Tunnels (14) einer Vorrichtung (11) nach Anspruch 1, umfassend die folgenden Schritte:
- Bereitstellen der sterilen Abdeckung (2, 2') mit zylindrischer Form an einem ersten axialen Ende des Tunnels (14) und
- sowohl Entfalten als auch Andrücken der sterilen Abdeckung (2, 2') an die Innenwand des Tunnels (14), indem das schirmartige Mittel (6) durch die sterile Abdeckung (2, 2') in dem Tunnel (14) geschoben wird.

## Revendications

1. Dispositif (11) d'imagerie d'au moins une partie d'un patient (15), comprenant
- des moyens (13) de détection comprenant un tunnel (14), dans lequel le patient (15) peut être transporté à des fins d'imagerie,
- une couverture (2, 2') stérile enroulée ou pliée à l'extérieur du tunnel (14) et
- un mécanisme (3) de recouvrement apte à dérouler ou à déplier la couverture (2, 2') stérile et à la draper dans le tunnel (14), de manière à ce que la paroi intérieure du tunnel (14) soit recouverte au moins en partie de la couverture (2, 2') stérile,
**caractérisé en ce que**
le mécanisme (3) de recouvrement comprend des moyens (6) semblables à un parapluie pour déplier la couverture (2, 2') stérile et pour presser la couverture (2, 2') stérile sur la paroi intérieure du tunnel (14), lorsque le moyen (6) analogue a un parapluie est poussé dans la couverture (2, 2') stérile dans le tunnel (14).

2. Dispositif (11) suivant la revendication 1, dans lequel le moyen de détection repose sur une technologie TRM ou sur une technologie CT.

3. Dispositif (11) suivant la revendication 1 ou 2, comprenant en outre une table (16) pour transporter le patient (15) dans le tunnel (14), dans lequel la table (16) peut être déplacée dans le couvercle (14) en étant recouverte de la couverture (2, 2') stérile sans l'endommager.

4. Dispositif (11) suivant l'une des revendications précédentes, dans lequel la couverture (2, 2') stérile a une forme cylindrique ayant un diamètre de l'ordre de celui du tunnel (14).

5. Dispositif (11) suivant la revendication 4, dans lequel la couverture (2, 2') stérile est l'une d'une série de couvertures, chacune séparée de l'autre par une perforation (4) respective.

6. Dispositif (11) suivant l'une des revendications précédentes, dans lequel la couverture (2, 2') stérile et/ou la paroi intérieure du tunnel (14) comprend du matériau collant pour fixer la couverture (2, 2') stérile à la paroi intérieure du tunnel (14).

7. Dispositif (11) suivant l'une des revendications précédentes, dans lequel la couverture (2, 2') stérile est faite en grande partie de papier ou d'un film en matière plastique.

8. Système pour effectuer une intervention guidée par une image, comprenant un dispositif (11) d'imagerie suivant l'une des revendications précédentes et un dispositif d'intervention.

9. Procédé pour recouvrir un tunnel (14) d'un dispositif (11) suivant la revendication 1, comprenant les stades de
- mettre la couverture (2, 2') stérile de forme cylindrique à une première extrémité axiale du tunnel (14) et
- à la fois déplier et presser la couverture (2, 2') stérile sur la paroi intérieure du tunnel (14) en poussant le moyen (6) analogue à un parapluie dans la couverture (2, 2') stérile dans le tunnel (14).
